# EUROPEAN PATENT SPECIFICATION

(11) **EP 4 487 825 B1**
(45) Date of publication and mention of the grant of the patent: **02.12.2026**
(21) Application number: 24197747.9
(22) Date of filing: 12.09.2018
(51) Int. Cl.: A61F 2/95, A61F 2/966

(54) **LOW PROFILE DELIVERY SYSTEM WITH LOCK WIRE LUMEN**
NIEDRIGPROFILIGES ABGABESYSTEM MIT VERRIEGELUNGSDRAHTLUMEN
SYSTÈME D'ADMINISTRATION À BAS PROFIL AVEC LUMIÈRE DE FIL-FREIN

(43) Date of publication of application: 08.01.2025
(62) Document number(s) of the earlier application(s) in accordance with Art. 76 EPC: 18779918.4 / 3 849 477
(73) Proprietor: W. L. Gore & Associates, Inc., Newark, DE 19711 (US)
(72) Inventor: BURKART, Dustin C., Newark, 19711 (US)
(74) Representative: HGF

(56) References cited:
- EP-A1- 2 777 643
- WO-A2-2009/042789
- US-A1- 2006 095 119

## Description

### FIELD

The present invention relates to medical devices for treating an anatomical space (e.g., vessels) of the body. More specifically, the invention relates to apparatuses, and systems that include an implantable medical device prosthesis that allows for accurate deployment in the anatomical space.

### BACKGROUND

Disease of the vasculature is increasingly common. Treatment of the vasculature may be difficult because of the tortuous nature and complexity of the vasculature. Aortic dissections, for example, commonly begin at or near the aortic valve root and continue to the ascending aorta and the aortic arch. Medical devices implanted at a diseased state may be used for treatment of aortic dissections, aneurysms, and other diseases of the vasculature or other luminal systems of the body, such as the biliary tract, gastrointestinal tract, or respiratory system, for example.

EP 2777643 is directed to delivery device for deploying an expandable, endoluminal prosthesis within a body vessel. The delivery device may include one or more stent graft retention scaffolds having a plurality of arches, each arch encompassing an aperture forming a helical path and a trigger wire passing through at least two of the apertures following the helical path, wherein at least a portion of the stent graft is retained in a compressed configuration on the delivery device by the retention scaffold and trigger wire.

It remains desirable to provide medical devices, systems and methods for repairing disease along the aorta and also for repairing disease along the aorta and the branches extending and luminal spaces therefrom.

### SUMMARY

The invention is defined by the appended claims.

In one example ("Example 1"), a medical device delivery system includes a catheter shaft; at least one loop attached to the catheter shaft; a medical device having an interior and an exterior; at least one opening through the medical device configured to allow the loop to extend from the interior to the exterior of the medical device; and a release line exterior to the medical device held in place by passing through the loop.

In another example ("Example 2"), further to the system of Example 1, the at least one loop is flexible and includes at least two flexible loops aligned relative to a longitudinal axis of the medical device.

In another example ("Example 3"), further to the system of any one of Examples 1-2, the at least one flexible loop includes a fiber coupled to the catheter shaft.

In another example ("Example 4"), further to the system of Example 3, the fiber is at least one of wrapped, glued, and bonded to the catheter shaft to form the at least one flexible loop.

In another example ("Example 5"), further to the system of any one of Examples 1-4, the medical device is configured to collapse to a delivery configuration and the at least one flexible loop is configured to collapse toward the catheter shaft in response to the medical device being collapse to the delivery configuration.

In another example ("Example 6"), further to the system of Example 5, the system also includes at least one sheath configured to releasably hold the medical device in the delivery configuration.

In another example ("Example 7"), further to the system of any one of Examples 1-6, the medical device comprises a stent and graft combination and the at least one opening is formed through the graft, and the at least one flexible loop comprises a graft material.

In another example ("Example 8"), further to the system of any one of Examples 1-7, the at least one flexible loop is configured to maintain at least a portion of the release line in contact with an exterior surface of the medical device.

In another example ("Example 9"), further to the system of any one of Examples 1-8, the at least one flexible loop is configured to form a lumen for the release line.

In another example ("Example 10"), further to the system of any one of Examples 1-9, the release line terminates in an olive at one end of the catheter shaft.

In another example ("Example 11"), further to the system of Example 10, the release line is configured to release from the olive in response to tension and withdraw through the at least one flexible loop.

In another example ("Example 12"), further to the system of any one of Examples 1-11, the release line and the at least one loop are configured to maintain the medical device coupled to the catheter shaft during orientation of the medical device.

In one example ("Example 13"), a method of manufacturing a delivery system for a medical device includes coupling at least one flexible loop to a catheter shaft; arranging the at least one flexible loop through an opening in the medical device; and arranging a release line through the at least one flexible loop to releasably couple the medical device to the catheter shaft.

In another Example ("Example 14"), further to the method of Example 13, coupling the at least one flexible loop to the catheter shaft includes at least one of wrapping, gluing, and bonding a fiber to the catheter shaft to form the at least one flexible loop.

In another example ("Example 15"), further to the method of any one of Examples 13-14, the method also includes collapsing the medical device against the catheter shaft to a delivery configuration and collapsing the at least one flexile loop toward the catheter shaft in response to the medical device being collapse to the delivery configuration.

In another example ("Example 16") a medical device delivery system includes a catheter shaft; a medical device having an interior and an exterior; a flexible lumen attached to the catheter shaft and extending through at least one opening through the medical device, the flexible lumen being configured to collapse in response to reduction in diameter of the medical device; and a release line arranged through the flexible lumen and configured to releasably couple the medical device to the catheter shaft.

In another example ("Example 17"), further to the system of Example 16, the flexible lumen includes a first flexible loop and a second flexible loop arranged at opposite ends of the medical device.

In another example ("Example 18"), further to the system of Example 17, the release line is configured to withdrawn through the first flexible loop and the second flexible loop in response to tension, and the first flexible loop and the second flexible loop maintain coupled to the catheter shaft.

In another example ("Example 19"), further to the system of Example 18, the catheter shaft is configured to withdraw the first flexible loop and the second flexible loop after delivery of the medical device.

In another example ("Example 20"), further to the system of Example 16, the at least one flexible loop includes a fiber coupled to the catheter shaft and the fiber is at least one of wrapped, glued, and bonded to the catheter shaft to form the at least one flexible loop.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 illustrates a side view of an example delivery system having a medical device in accordance with an embodiment.
FIG. 2 illustrates a perspective view of an example delivery system having a medical device in accordance with an embodiment.
FIG. 3 illustrates a perspective view of an example release line and a medical device in accordance with an embodiment.
FIG. 4 illustrates a side view of an example release line and a catheter shaft in accordance with an embodiment.
FIG. 5 illustrates a close-up view of an example flexible loop, a medical device, and a catheter shaft in accordance with an embodiment.
FIG. 6 illustrates a close-up view of an example flexible loop and a catheter shaft in accordance with an embodiment.
FIG. 7 illustrates a side view of an example release line, flexible loop, and a catheter shaft in accordance with an embodiment.

### DETAILED DESCRIPTION

Persons skilled in the art will readily appreciate that various aspects of the present disclosure can be realized by any number of methods and apparatus configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward apparatuses, systems, and methods that include an implantable medical device that may be used in treatment of the vasculature or other luminal and non-luminal systems of the body. In some examples, the implantable medical device is delivered to the vasculature using a delivery system, such as a transcatheter delivery system. In terms of configurations for the implantable medical device, a variety of configurations are contemplated, and the implantable medical devices described herein may be substantially cylindrical, include a bifurcation, or have any of a variety of features. Further, the implantable medical devices may be configured to conform to the vasculature into which the implantable medical device is implanted, and have a profile that facilitates delivery of the implantable medical device using a minimally invasive procedure (e.g., via transcatheter techniques), and able to withstand forces and other stresses that occur once implanted in the vasculature.

The medical device may be collapsed to a delivery configuration for delivery and expanded at the target location. In certain instances, the medical device may be partially expanded (e.g., for steering or orienting) or remain otherwise coupled to the catheter shaft during deployment. To maintain coupling of the medical device to the catheter, the delivery system may include a release line coupled to the medical device and the catheter.

As compared to delivery systems without a structural aspect to releasably couple a medical device to a catheter shaft, the release line, in certain instances, can increase an overall delivery profile of the system due in part to a catheter lumen for the release line. To facilitate delivery of the medical device with a release line (or other similar device-to-catheter coupling), the systems discussed herein include a flexible loop or lumen through which the release line is arranged. As discussed in further detail below, the flexible loop or lumen facilitate delivery of the medical devices discussed herein.

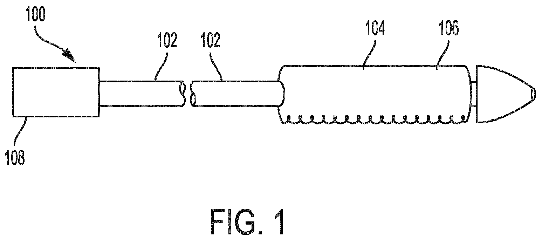

FIG. 1 illustrates a side view of an example delivery system 100 having a medical device 106 in accordance with an embodiment. The medical device 106 may be any endoluminal device suitable for delivery to the treatment area of a vasculature, or other luminal and non-luminal systems of the body (biliary tract, GI tract, respiratory system, or other as desired). As shown in FIG. 1, the delivery system 100 may also include a handle 108 at a proximal end of the system (the opposite end of the system at which the medical device 106 is arranged). The handle 108 of the delivery system 100 may be accessible to a user to assist in delivering the medical device 106. As explained in further detail below, the delivery system 100 may include one or more lines or wires that facilitate delivery, orientation, or placement of the medical device 106.

In various embodiments, the medical device 106 can include a radially collapsed configuration suitable for delivery to the treatment area of the vasculature of a patient. The medical device 106 can be constrained toward a radially collapsed configuration and releasably mounted onto a delivery device such as catheter shaft 102. The diameter of the medical device 106 in the collapsed configuration is small enough for the medical device 106 to be delivered through the vasculature to a target treatment location or area. In various embodiments, the diameter of the collapsed configuration is small enough to minimize the crossing profile of the delivery system 100 and reduce or prevent tissue damage to the patient. In the collapsed configuration, the medical device 106 can be guided by a catheter shaft 102 through the vasculature.

The medical device 106 can comprise a radially expanded configuration suitable for implanting the medical device 106 in the treatment area of a patient's vasculature. In the expanded configuration, the diameter of the medical device 106 can be approximately the same as the vessel or other body lumen in which the medical device 106 is to be placed. In other embodiments, the diameter of the medical device 106 in the expanded configuration can be slightly larger than the vessel to be treated to provide a friction or interference fit within the vessel.

In various embodiments, medical device 106 can comprise a self-expandable device, such as a self-expandable stent graft. Such devices dilate from a radially collapsed configuration to a radially expanded configuration when unrestrained. In other embodiments, medical device 106 can comprise a device that is expanded with the assistance of a secondary device applying an expansion force on the medical device 106, such as, for example, a balloon. In yet other embodiments, delivery system 100 can comprise a plurality of medical devices 106. The use of the delivery system 100 with any number of medical devices 106 is within the scope of the present disclosure. The medical devices 106 discussed herein may include, for example, stents, grafts, stent grafts, heart valves, and other similar devices. Thus, medical device 106 can include one or more stent components with one or more graft members disposed over and/or under the stent, which can dilate from a delivery diameter, through a range of larger intermediary diameters, and toward a maximal, pre-determined functional diameter. In certain instances, the medical device 106 includes one or more stent components made of nitinol and a graft member made of ePTFE. However, and as discussed below, any suitable combination of stent component(s) and graft member(s) is within the scope of the present disclosure.

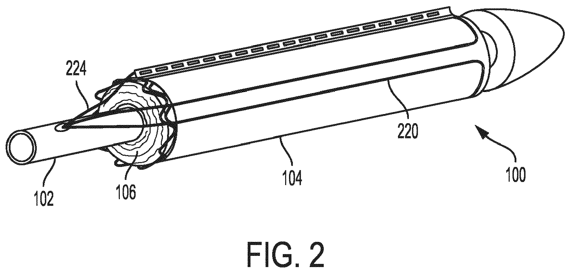

In certain instances, the delivery system 100 may include a sleeve or multiple sleeves (and other constrain mechanisms) to constrain the medical device 106 in a collapsed configuration for endoluminal delivery of the medical device 106 to a treatment portion of the vasculature or other body lumen of a patient. For the purposes of the disclosure, the term "constrain" may mean (i) to limit the expansion, either through self-expansion or assisted by a device, of the diameter of a medical device 106 or (ii) to cover or surround but not otherwise restrain a medical device 106 (e.g., for storage or biocompatibility reasons and/or to provide protection to the medical device 106 and/or the vasculature). The delivery system 100, for example, comprises a sleeve 104 (or pull-back sheath or other similar constraining structure) which surrounds and constrains medical device 106 toward a reduced diameter or collapsed configuration as shown in further detail with reference to FIG. 2.

FIG. 2 illustrates a perspective view of an example delivery system 100 having a medical device 106 in accordance with an embodiment. In various embodiments, the medical device 106 is constrained by a single sleeve 104 which circumferentially surrounds the medical device 106. In various embodiments, the sleeve 104 circumferentially surrounds the medical device 106 and constrains it toward a collapsed configuration, in which the diameter is less than the diameter of the medical device 106 in an unconstrained or otherwise deployed state or configuration. For example, the sleeve 104 may constrain the medical device 106 toward a collapsed configuration for delivery within the vasculature.

In other embodiments, the medical device 106 is constrained by a plurality of sleeves (e.g., similar to the sleeve 104) which circumferentially surround the medical device 106, which allow the medical device 106 to be deployed and held at intermediate configurations larger than the collapsed configuration and smaller than the deployed configuration. The plurality of sleeves can comprise at least two sleeves (e.g., each similar to sleeve 104) which circumferentially surround each other.

The sheet of material(s) used to form the sleeve(s) can comprise a series of openings, such that the openings extend from one edge of the sheet to the other. In such configurations, a coupling member 224 can be woven or stitched through the series of openings in the sheet of material(s), securing each of the two edges together and forming a tube. For example, in FIG. 2, the coupling member 224 secures the edges of sleeve 104 such that sleeve 104 maintains medical device 106 toward a reduced diameter or outer peripheral dimension suitable for endoluminal delivery.

In various embodiments, disengaging a single coupling member which closes a single sleeve from the sleeve allows the expandable device to be expanded toward a larger diameter or outer peripheral dimension. For example, with reference to FIG. 2, the delivery system can be used to deliver the medical device 106 to a treatment area of a vasculature. The medical device 106 has a collapsed diameter for delivery, and sleeve 104 circumferentially surrounds the medical device 106 and is held closed by the coupling member 224. As described in more detail below, bending of the medical device 106 can be controlled prior to full expansion (e.g., at an intermediate diameter) to help facilitate delivery to the desired position. Once medical device 106 is in position relative to the treatment area, the coupling member 224 is disengaged from the sleeve 104 and the sleeve 104 is released, allowing medical device 106 to expand toward a larger diameter.

In such embodiments, the medical device 106 can be expanded from the collapsed configuration toward the intermediate configuration once the implant has been delivered near the treatment area of the vasculature of a patient. The intermediate configuration may, among other things, assist in properly orienting and locating the medical device 106 within the treatment area of the vasculature. In various embodiments, a medical device 106 can be concentrically surrounded by two sleeves having different diameters. In such configurations, a primary sleeve constrains the medical device 106 toward the collapsed configuration. Once the collapsed configuration sleeve is opened, a secondary sleeve constrains the medical device 106 toward the intermediate configuration similarly configured and arranged as the sleeve 104 (e.g., the secondary sleeve may also be held together released by a coupling member).

As noted above, the medical device 106 may be oriented at the treatment area of the vasculature. To orient the medical device 106, the delivery system 100 includes a steering line 220. Tension can be applied to the steering line 220 to displace the steering line 220 and bend the medical device 106. Bending the medical device 106 may, among other things, assist in travelling through curved or tortuous regions of vasculature. Bending the medical device 106 may also allow the medical device 106 to conform to curvatures in the vasculature, or other body lumens of a patient.

In certain instances, the delivery system 100 may include a handle 108 (as shown in FIG. 1) to which the coupling member 224 and the steering line 220 (or lines) are connected. The handle 108 may allow the user to manipulate the coupling member 224 and the steering line 220 outside of the patient.

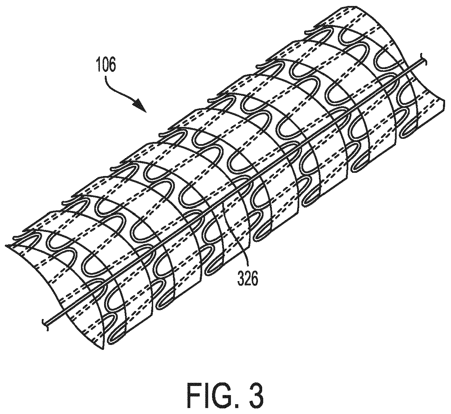

FIG. 3 illustrates a perspective view of an example release line 326 and a medical device 106 in accordance with an embodiment. A delivery system 100 may include a release line 326 to the medical device 106 to the delivery system 100. In addition, the release line 326 may also be used to secure a steering line 220 or multiple steering lines (e.g., each similar to another as desired) to the delivery system 100.

The release line 326 may be arranged external to the medical device 106 (as shown), may be internal to the medical device 106, or may be routed through the medical device 106 such that portions of the release line 326 are external to the medical device 106 and other portions of the release line 326 are internal to the medical device 106. Another end of the release line 326, similar to the coupling member 224 and steering line 220 discussed above, may be coupled to a handle 108 (not shown) of the delivery system 100. At the handle 108, tension may be applied to the release line 326 by a user to remove the release line 326 from the patient and uncouple the medical device 106 from the delivery system 100.

In various embodiments, the release line 326 can be formed from metallic, polymeric or natural materials and can comprise conventional medical grade materials such as nylon, polyacrylamide, polycarbonate, polyethylene, polyformaldehyde, polymethylmethacrylate, polypropylene, polytetrafluoroethylene, polytrifluorochlorethylene, polyvinylchloride, polyurethane, elastomeric organosilicon polymers; metals such as stainless steels, cobalt-chromium alloys and nitinol. Elongated members or lock wires can also be formed from high strength polymer fibers such as ultra high molecular weight polyethylene fibers (e.g., Spectra^{®}, Dyneema Purity^{®}, etc.) or aramid fibers (e.g., Technora^{®}, etc.).

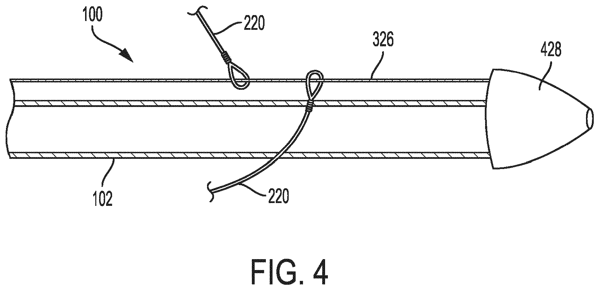

FIG. 4 illustrates a side view of an example release line 326 and a catheter shaft 102 in accordance with an embodiment. As shown in FIG. 4, a delivery system 100 incudes a catheter shaft 102, medical device 106, steering lines 220, and a release line 326. The release line 326 passes from outside of the body of the patient, from a handle 108 (not shown), through the catheter shaft 102, and exits distal to or near a distal end of the medical device 106. At this point, the release line 326 may extend along an exterior surface of the medical device 106.

In certain instances and as shown in FIG. 4, the release line 326 interacts with steering lines 220 continues to catheter tip 428. In such a configuration, the release line 326 releasably couples steering lines 220 to the delivery system 100. The release line 326 may be embedded, affixed, or attached to the catheter tip 428, and may be released from the catheter tip 428 in response to tension applied to the release line 326 by a user. Any manner in which the release line 326 may interact with steering line or lines 220 to maintain a releasable coupling between steering line or lines 220 and the delivery system 100 is within the scope of the present disclosure. Although two steering lines 220 are shown, it should be understood any number of steering lines 220 is contemplated, including fewer or greater than the two shown in FIG. 4.

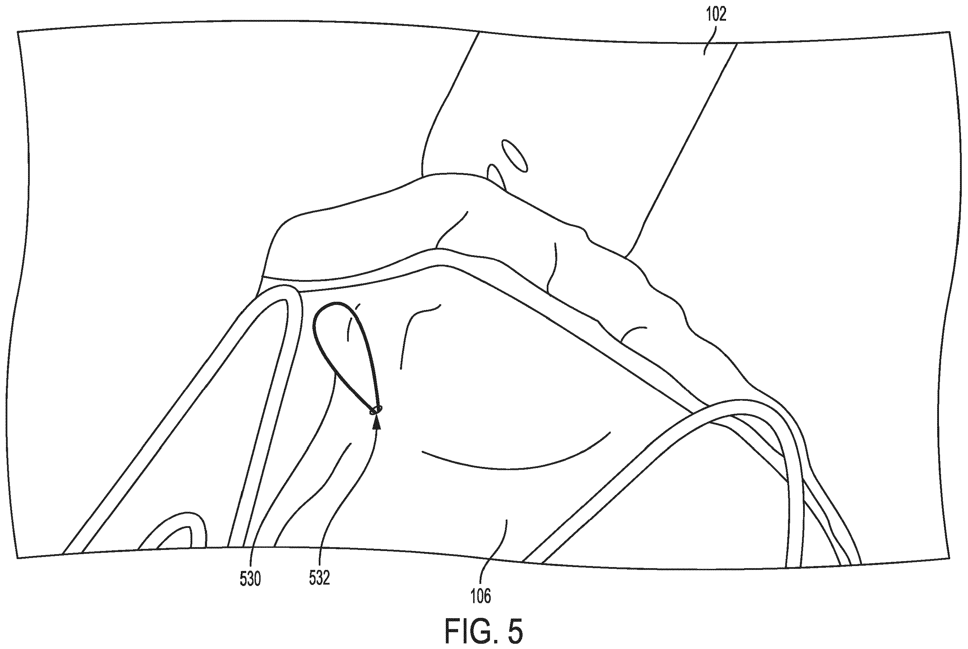

FIG. 5 illustrates a close-up view of a flexible loop 530, a medical device 106, and a catheter shaft 102 (that the medical device 106 is crushed or constrained onto) in accordance with an embodiment. As shown in FIG. 5, the medical device 106 includes an opening 532. The opening 532 through the medical device 106 may be configured to allow the flexible loop 530 to extend from an interior of the medical device 106 (through which the catheter shaft 102 is arranged) to an exterior of the medical device 106 as shown in FIG. 5. In certain instances, the opening 532 through the medical device 106 may be configured to allow the flexible loop 530 to extend from an exterior of the medical device 106 to an interior of the medical device 106.

The medical device 106 may be collapsed to a delivery configuration for delivery and expanded at the target location. In certain instances, the medical device 106 may be partially expanded (e.g., for steering or orienting) or remain otherwise coupled to the catheter shaft during deployment. To maintain coupling of the medical device 106 to the catheter shaft 102, the delivery system may include a release line 326 (as shown in FIGs. 3-4 and FIG. 7) coupled to the medical device 106 and the catheter shaft 102.

As compared to delivery systems without a structural aspect (e.g., a release wire, lock wire, or other similar structure) to releasably couple the medical device 106 to the catheter shaft 102, the release line 326, in certain instances, can increase an overall delivery profile of the system 100 due in part to the requirement for a catheter lumen to maintain the release line. To facilitate delivery of the medical device 106 with a release line (or other similar device-to-catheter coupling), a flexible loop 530 may serve as a pathway for the release line 326. In certain instances, the delivery systems discussed herein may include multiple flexible loops 530. In certain instances, the flexible loop 530 (or flexible loops 530) may form a flexible lumen through which the release line 326 is arranged.

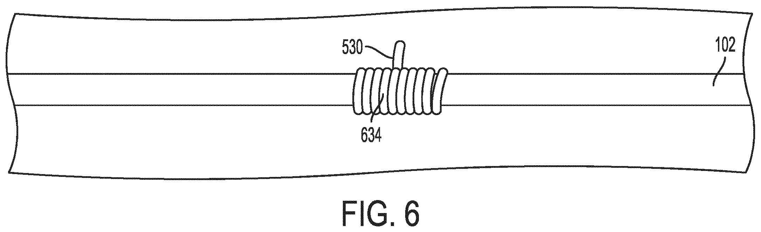

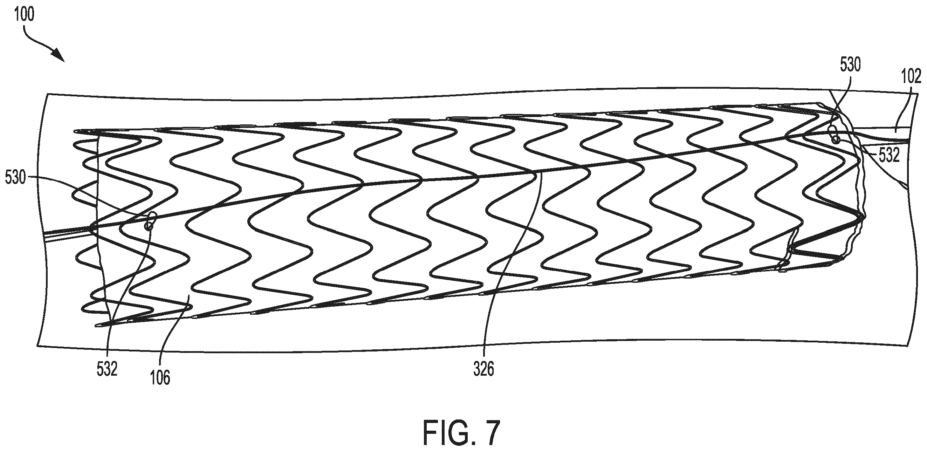

In certain instances, the flexible loop 530 (flexible loops 530) may be attached to the catheter shaft 102 (as shown in FIG. 6). The flexible loop 530 (flexible loops 530 or lumen) may be configured to collapse in response to reduction in diameter of the medical device 106. In this manner, the flexible loop 530 (flexible loops 530 or lumen) may facilitate delivery by forming a pathway or lumen for a release wire. Thus, the flexible loop 530 (flexible loops 530 or lumen) lessen a size or diameter of a catheter usable for the delivery systems discussed herein by forming a pathway for a release wire where a lumen of a catheter would previously been used. As shown in further detail in FIG. 7, the flexible loop 530 (flexible loops 530 or lumen) may hold the release line 326 or similar feature in place exterior to the medical device 106 by being passed through the flexible loop 530.

FIG. 6 illustrates a close-up view of an example flexible loop 530 and a catheter shaft 102 in accordance with an embodiment. As shown in FIG. 6, the flexible loop 530 is coupled to the catheter shaft 102. The flexible loop 530 may be attached to the catheter shaft 102 along a portion of the catheter shaft 102 that extends through a lumen of a medical device, such as the medical device 106.

In certain instances, the flexible loop 530 includes a fiber 634 coupled to the catheter shaft 102. The fiber 634 may be wrapped, glued, bonded, sewn, tacked, interlocked or otherwise coupled to the catheter shaft 102 to form the flexible loop 530. The medical device 106 (as discussed above but not shown) discussed herein may be a stent and graft combination, or any configuration described in association with the medical device 106. The flexible loop 530 may be formed of the same or a similar material as the graft material of the medical device. In addition, the opening through which the flexible loop 530 is arranged may be arranged through the graft portion of the medical device 106.

To manufacture a delivery system that includes the flexible loop 530 or more than one flexible loop 530, the flexible loop 530 is coupled to the catheter shaft 102 (e.g., wrapping, gluing, bonding, sewing, tacking, interlocking, or otherwise coupling the fiber 634 to the catheter shaft 102 to form the flexible loop 530). After the flexible loop 530 is formed, the flexible loop 530 is arranged through an opening (e.g., as shown in FIG. 5) in the medical device 106. Subsequently, a release line may be arranged through the flexible loop 530 to releasably couple the medical device to the catheter shaft 102 (e.g., as shown in FIG. 7). After the release line is arranged through the flexible loop 530, the medical device may be collapsed against the catheter shaft 102 to a delivery configuration, which can also include collapsing of the flexile loop 530 toward the catheter shaft 102 in response to the medical device being collapsed to the delivery configuration.

FIG. 7 illustrates a side view of an example release line 326, flexible loop 530, and a catheter shaft 102 in accordance with an embodiment. The release line 326, flexible loop 530, and catheter shaft 102 form portions of a delivery system 100. The medical device 106 has an interior and an exterior. As shown in FIG. 7, a portion of the catheter shaft 102 is arranged through the interior of the medical device 106, and the release line 326 is arranged across the exterior of the medical device 106.

As shown in FIG. 7, the delivery system 100 includes two flexible loops 530. In certain instances, the two flexible loops 530 are aligned relative to a longitudinal axis of the medical device 106, which may be aligned with the catheter shaft 102. Further, the medical device 106 is configured to collapse to a delivery configuration from an expanded/delivery configuration shown in FIG. 7. In these instances, the flexible loop 530 or flexible loops 530 are configured to collapse toward the catheter shaft 102 in response to the medical device 106 being collapsed to the delivery configuration. The delivery system 100 may include one or more sheaths/sleeves (e.g., as shown in FIGs. 1-2) configured to releasably hold the medical device 106 in the delivery configuration.

In certain instances, the flexible loop 530 (or loops 530) is configured to maintain at least a portion of the release line 326 in contact with an exterior surface of the medical device 106. Thus, the flexible loop 530 is configured to form a lumen for the release line 326 in certain instances. The flexible loop 530 (or flexible loops 530 as shown in FIG. 7) are attached to the catheter shaft 102 and extend through an opening 532 (or opening 532 for each respective flexible loop 530) through the medical device 106. The flexible loop 530 (or lumen) is configured to collapse in response to reduction in diameter of the medical device 106. In certain instances, the lumen is formed by a first loop 530 arranged at one of the medical device 106 and a second loop 530 arranged at another end of the medical device 106, with the release line 326 being arranged through the flexible lumen (formed by the loops 530). The release line 326 is configured to releasably couple the medical device 106 to the catheter shaft 102.

In certain instances and as shown in FIG. 4, the release line 326 may terminate at a catheter tip 428 (or olive) of the catheter shaft 102. The release line 326 is configured to release from the catheter tip 428 (or olive) in response to tension and withdraw through the flexible loop(s) 530. As noted above, the medical device 106 may be collapsed to a delivery configuration for delivery and expanded at the target location. In certain instances, the medical device 106 may be partially expanded (e.g., for steering or orienting) or remain otherwise coupled to the catheter shaft during deployment. To maintain coupling of the medical device 106 to the catheter shaft 102, the release line 326 is coupled to the medical device 106 by being arranged through the flexible loop(s) 530 (or lumen) and to the catheter shaft 102. Thus, the medical device 106 may be partially expanded and maintained in contact with the delivery system 100 and the catheter shaft 102 by way of the release line 326. The release line 326 and the loop(s) 539 are configured to maintain the medical device 106 coupled to the catheter shaft 102 during orientation of the medical device 106.

The flexible loop(s) 530, in certain instances, facilitate a minimal delivery profile of the system 100 due in part to removing the need for a lumen for the catheter lumen for the release line 326 (which can add to overall delivery size). In certain instances, the flexible loop 530 (or flexible loops 530) may form a flexible lumen through which the release line 326 is arranged, and which may collapse along with the medical device 106.

After orienting and deployment of the medical device 106, the release line 326 is configured to withdrawn through the flexible loop(s) 530 in response to tension. The flexible loop(s) 530 are fixedly coupled to the catheter shaft 106. The catheter shaft 102 may be removed from the vasculature after deployment of the medical device 106 and leave the medical device 106 at the target treatment location. The catheter shaft 102 may be withdrawn to remove the system 100 from the vasculature. By withdrawing the catheter shaft 102, the flexible loop(s) 530 are also removed due to the flexible loop(s) 530 being fixedly attached to the catheter shaft 102. The flexible loop(s) facilitate use of the release line 326 without increasing the size of the delivery system 100, which may facilitate use of the system 100 through reach into smaller vasculature as compared to prior systems.

Potential materials for graft members of the medical devices discussed herein include, for example, expanded polytetrafluoroethylene (ePTFE), polyester, polyurethane, fluoropolymers, such as perflouorelastomers and the like, polytetrafluoroethylene, silicones, urethanes, ultra-high molecular weight polyethylene, aramid fibers, and combinations thereof. Other embodiments for a graft member material can include high strength polymer fibers such as ultra-high molecular weight polyethylene fibers (e.g., Spectra^{®}, Dyneema Purity^{®}, etc.) or aramid fibers (e.g., Technora^{®}, etc.). The graft member may include a bioactive agent. In one embodiment, an ePTFE graft includes a carbon component along a blood contacting surface thereof. Any graft member which can be delivered by a catheter is in accordance with the present disclosure.

In addition, stent components of the medical devices discussed herein can have various configurations such as, for example, rings, cut tubes, wound wires (or ribbons) or flat patterned sheets rolled into a tubular form. Stent components can be formed from metallic, polymeric or natural materials and can comprise conventional medical grade materials such as nylon, polyacrylamide, polycarbonate, polyethylene, polyformaldehyde, polymethylmethacrylate, polypropylene, polytetrafluoroethylene, polytrifluorochlorethylene, polyvinylchloride, polyurethane, elastomeric organosilicon polymers; metals such as stainless steels, cobalt-chromium alloys and nitinol and biologically derived materials such as bovine arteries/veins, pericardium and collagen. Stent components can also comprise bioresorbable materials such as poly(amino acids), poly(anhydrides), poly(caprolactones), poly(lactic/glycolic acid) polymers, poly(hydroxybutyrates) and poly(orthoesters). Any expandable stent component configuration which can be delivered by a catheter is in accordance with the present disclosure.

The invention of this application has been described above both generically and with regard to specific embodiments. It will be apparent to those skilled in the art that various modifications and variations can be made in the embodiments without departing from the scope of the disclosure as long as they are encompassed by the claims. Thus, it is intended that the embodiments cover the modifications and variations of this invention provided they come within the scope of the appended claims.

## Claims

1. A medical device delivery system, the system comprising:
a catheter shaft, (102)
a medical device (106) coupled to the catheter shaft in a delivery configuration, the medical device having an interior and an exterior and at least one opening (532) defined between the interior and the exterior;
at least one flexible loop extending from the interior to the exterior and through the opening of the medical device and attached to the catheter shaft; (102) and
a release line (326) exterior to the medical device held in place by extending through the at least one flexible loop, the release line aligned relative to a longitudinal axis of the medical device,
wherein the at least one flexible loop is configured to maintain at least a portion of the release line in contact with an exterior surface of the medical device.

2. The medical device delivery system of claim 1, wherein the medical device is configured to collapse to the delivery configuration, and when the medical device collapses, the at least one flexible loop is configured to collapse toward the catheter shaft.

3. The medical delivery system of claim 1, further comprising at least one sheath (104) configured to releasably hold the medical device in the delivery configuration.

4. The medical delivery system of claim 3, wherein the at least one sheath includes a first sheath and a second sheath positioned under the first sheath, wherein when the first sheath is open, the second sheath constrains the device to an intermediate configuration between the delivery configuration and an expanded configuration.

5. The medical delivery system of claim 1, wherein the at least one flexible loop includes a fiber; (634) and optionally wherein the fiber is coupled to the catheter shaft by at least one of wrapping, gluingor bonding to form the at least one flexible loop.

6. The medical delivery system of claim 1, wherein the medical device comprises a stent and graft combination and the at least one opening is formed through the graft; and optionally wherein the at least one flexible loop comprises a material that is the same or similar to the graft material.

7. The medical delivery system of claim 1, wherein the release line terminates in an olive at one end of the catheter shaft; and optionally wherein the release line is configured to release from the olive in response to tension and withdraw through the at least one flexible loop.

8. The medical delivery system of claim 1, wherein the release line and the at least one loop are configured to maintain the medical device coupled to the catheter shaft during orientation of the medical device.

9. The medical delivery system of claim 1, wherein the at least one loop includes at least two flexible loops aligned relative to a longitudinal axis of the medical device such that the release line extends along a portion of the longitudinal axis of the medical device.

10. A method of manufacturing a delivery system for a medical device, the method comprising:
arranging the at least one flexible loop through an opening in the medical device, the at least one flexible loop being coupled to a catheter shaft of the delivery system; and
arranging a release line through the at least one flexible loop to hold the release line in place and releasably couple the medical device to the catheter shaft;
wherein the release line extends parallel relative to a longitudinal axis of the delivery system and exterior to the medical device; and
wherein the at least one flexible loop is configured to maintain at least a portion of the release line in contact with an exterior surface of the medical device.

11. The method of claim 10, wherein the at least one flexible loop includes a fiber, the method including at least one of wrapping, gluing, and bonding to couple the fiber to the catheter shaft to form the at least one flexible loop.

12. The method of claim 10, wherein the at least one loop includes at least two flexible loops, the method including arranging the release line through the at least two flexible loops.

13. The method of claim 12, including arranging a first loop of the at least two flexible loops at a first end of the medical device and arranging a second loop of the at least two flexible loops at a second end of the medical device.

14. The method of claim 10, further including collapsing of the flexible loop toward the catheter shaft in response to the medical device being collapsed to the delivery configuration against the catheter shaft.

## Patentansprüche

1. Abgabesystem für medizinische Vorrichtungen, wobei das System Folgendes umfasst:
einen Katheterschaft, (102)
eine medizinische Vorrichtung (106), die in einer Abgabekonfiguration an den Katheterschaft gekoppelt ist, wobei die medizinische Vorrichtung ein Inneres und ein Äußeres und mindestens eine Öffnung (532) aufweist, die zwischen dem Inneren und dem Äußeren definiert ist, wobei mindestens eine flexible Schlaufe von dem Inneren zu dem Äußeren und durch die Öffnung der medizinischen Vorrichtung verläuft und an dem Katheterschaft befestigt ist; (102) und
eine Freigabeleitung (326) außerhalb der medizinischen Vorrichtung, die durch Verlaufen durch die mindestens eine flexible Schlaufe an Ort und Stelle gehalten wird, wobei die Freigabeleitung relativ zu einer Längsachse der medizinischen Vorrichtung ausgerichtet ist,
wobei die mindestens eine flexible Schlaufe dazu konfiguriert ist, mindestens einen Abschnitt der Freigabeleitung in Kontakt mit einer Außenfläche der medizinischen Vorrichtung zu halten.

2. Abgabesystem für medizinische Vorrichtungen nach Anspruch 1, wobei die medizinische Vorrichtung dazu konfiguriert ist, in die Abgabekonfiguration zusammenzuklappen, und wenn die medizinische Vorrichtung zusammenklappt, die mindestens eine flexible Schlaufe dazu konfiguriert ist, zu dem Katheterschaft hin zusammenzuklappen.

3. Medizinisches Abgabesystem nach Anspruch 1, ferner umfassend mindestens eine Hülle (104), die dazu konfiguriert ist, die medizinische Vorrichtung lösbar in der Abgabekonfiguration zu halten.

4. Medizinisches Abgabesystem nach Anspruch 3, wobei die mindestens eine Hülle eine erste Hülle und eine zweite Hülle beinhaltet, die unter der ersten Hülle positioniert ist, wobei, wenn die erste Hülle offen ist, die zweite Hülle die Vorrichtung auf eine Zwischenkonfiguration zwischen der Abgabekonfiguration und einer erweiterten Konfiguration beschränkt.

5. Medizinisches Abgabesystem nach Anspruch 1, wobei die mindestens eine flexible Schlaufe eine Faser beinhaltet; (634) und wobei optional die Faser an den Katheterschaft durch mindestens eines von Umwickeln, Kleben oder Binden gekoppelt ist, um die mindestens eine flexible Schlaufe zu bilden.

6. Medizinisches Abgabesystem nach Anspruch 1, wobei die medizinische Vorrichtung eine Kombination aus Stent und Transplantat umfasst und die mindestens eine Öffnung durch das Transplantat gebildet ist; und wobei optional die mindestens eine flexible Schlaufe ein Material umfasst, das dem Transplantatmaterial gleich oder ähnlich ist.

7. Medizinisches Abgabesystem nach Anspruch 1, wobei die Freigabeleitung in einer Olive an einem Ende des Katheterschafts endet; und wobei optional die Freigabeleitung dazu konfiguriert ist, sich von der Olive als Reaktion auf Spannung zu lösen und sich durch die mindestens eine flexible Schlaufe zurückzuziehen.

8. Medizinisches Abgabesystem nach Anspruch 1, wobei die Freigabeleitung und die mindestens eine Schlaufe dazu konfiguriert sind, die medizinische Vorrichtung an den Katheterschaft während der Orientierung der medizinischen Vorrichtung gekoppelt zu halten.

9. Medizinisches Abgabesystem nach Anspruch 1, wobei die mindestens eine Schlaufe mindestens zwei flexible Schlaufen beinhaltet, die relativ zu einer Längsachse der medizinischen Vorrichtung so ausgerichtet sind, dass die Freigabeleitung entlang eines Abschnitts der Längsachse der medizinischen Vorrichtung verläuft.

10. Verfahren zum Herstellen eines Abgabesystems für eine medizinische Vorrichtung, wobei das Verfahren Folgendes umfasst:
Anordnen der mindestens einen flexiblen Schlaufe durch eine Öffnung in der medizinischen Vorrichtung, wobei die mindestens eine flexible Schlaufe an einen Katheterschaft des Abgabesystems gekoppelt ist; und
Anordnen einer Freigabeleitung durch die mindestens eine flexible Schlaufe, um die Freigabeleitung an Ort und Stelle zu halten und die medizinische Vorrichtung lösbar an den Katheterschaft zu koppeln;
wobei die Freigabeleitung parallel relativ zu einer Längsachse des Abgabesystems und außerhalb der medizinischen Vorrichtung verläuft; und
wobei die mindestens eine flexible Schlaufe dazu konfiguriert ist, mindestens einen Abschnitt der Freigabeleitung in Kontakt mit einer Außenfläche der medizinischen Vorrichtung zu halten.

11. Verfahren nach Anspruch 10, wobei die mindestens eine flexible Schlaufe eine Faser beinhaltet, wobei das Verfahren mindestens eines von Umwickeln, Kleben und Binden beinhaltet, um die Faser an den Katheterschaft zu koppeln, um die mindestens eine flexible Schlaufe zu bilden.

12. Verfahren nach Anspruch 10, wobei die mindestens eine Schlaufe mindestens zwei flexible Schlaufen beinhaltet, wobei das Verfahren das Anordnen der Freigabeleitung durch die mindestens zwei flexiblen Schlaufen beinhaltet.

13. Verfahren nach Anspruch 12, beinhaltend das Anordnen einer ersten Schlaufe der mindestens zwei flexiblen Schlaufen an einem ersten Ende der medizinischen Vorrichtung und das Anordnen einer zweiten Schlaufe der mindestens zwei flexiblen Schlaufen an einem zweiten Ende der medizinischen Vorrichtung.

14. Verfahren nach Anspruch 10, ferner beinhaltend das Zusammenklappen der flexiblen Schlaufe zu dem Katheterschaft hin als Reaktion darauf, dass die medizinische Vorrichtung in die Abgabekonfiguration gegen den Katheterschaft zusammengeklappt wird.

## Revendications

1. Système d'administration de dispositif médical, le système comprenant :
une tige de cathéter (102),
un dispositif médical (106) couplé à la tige de cathéter dans une configuration d'administration, le dispositif médical possédant un intérieur et un extérieur et au moins une ouverture (532) définie entre l'intérieur et l'extérieur ;
au moins une boucle souple s'étendant de l'intérieur vers l'extérieur et à travers l'ouverture du dispositif médical et fixée à la tige de cathéter (102) ; et
une ligne de libération (326) extérieure au dispositif médical maintenue en place en s'étendant à travers ladite au moins une boucle souple, la ligne de libération étant alignée par rapport à un axe longitudinal du dispositif médical,
ladite au moins une boucle souple étant conçue pour maintenir au moins une partie de la ligne de libération en contact avec une surface extérieure du dispositif médical.

2. Système d'administration de dispositif médical selon la revendication 1, ledit dispositif médical étant conçu pour se replier vers la configuration d'administration, et lorsque le dispositif médical se replie, ladite au moins une boucle souple étant conçue pour se replier vers la tige de cathéter.

3. Système d'administration médicale selon la revendication 1, comprenant en outre au moins une gaine (104) conçue pour maintenir de façon amovible le dispositif médical dans la configuration d'administration.

4. Système d'administration médicale selon la revendication 3, ladite au moins une gaine comprenant une première gaine et une seconde gaine positionnée sous la première gaine, lorsque la première gaine est ouverte, ladite seconde gaine contraignant le dispositif vers une configuration intermédiaire entre la configuration d'administration et une configuration déployée.

5. Système d'administration médicale selon la revendication 1, ladite au moins une boucle souple comprenant une fibre (634) ; et éventuellement ladite fibre étant couplée à la tige de cathéter par au moins une opération parmi l'emballage, le collage ou la liaison de manière à former ladite au moins une boucle souple.

6. Système d'administration médicale selon la revendication 1, ledit dispositif médical comprenant une combinaison d'endoprothèse et de greffe et ladite au moins une ouverture étant formée à travers la greffe ; et éventuellement ladite au moins une boucle souple comprenant un matériau qui est identique ou similaire au matériau de greffe.

7. Système d'administration médicale selon la revendication 1, ladite ligne de libération se terminant par une olive à une extrémité de la tige de cathéter ; et éventuellement ladite ligne de libération étant conçue pour se libérer de l'olive en réponse à une tension et se retirer à travers ladite au moins une boucle souple.

8. Système d'administration médicale selon la revendication 1, ladite ligne de libération et ladite au moins une boucle étant conçues pour maintenir le dispositif médical couplé à la tige de cathéter pendant l'orientation du dispositif médical.

9. Système d'administration médicale selon la revendication 1, ladite au moins une boucle comprenant au moins deux boucles souples alignées par rapport à un axe longitudinal du dispositif médical de sorte que la ligne de libération s'étende le long d'une partie de l'axe longitudinal du dispositif médical.

10. Procédé de fabrication d'un système d'administration pour un dispositif médical, le procédé comprenant :
l'agencement de ladite au moins une boucle souple à travers une ouverture dans le dispositif médical, ladite au moins une boucle souple étant couplée à une tige de cathéter du système d'administration ; et
l'agencement d'une ligne de libération à travers ladite au moins une boucle souple pour maintenir la ligne de libération en place et coupler de manière amovible le dispositif médical à la tige de cathéter ;
ladite ligne de libération s'étendant parallèlement par rapport à un axe longitudinal du système d'administration et à l'extérieur du dispositif médical ; et
ladite au moins une boucle souple étant conçue pour maintenir au moins une partie de la ligne de libération en contact avec une surface extérieure du dispositif médical.

11. Procédé selon la revendication 10, ladite au moins une boucle souple comprenant une fibre, le procédé comprenant au moins une opération parmi l'emballage, le collage et la liaison pour coupler la fibre à la tige de cathéter de manière à former ladite au moins une boucle souple.

12. Procédé selon la revendication 10, ladite au moins une boucle comprenant au moins deux boucles souples, le procédé comprenant l'agencement de la ligne de libération à travers lesdites au moins deux boucles souples.

13. Procédé selon la revendication 12, comprenant l'agencement d'une première boucle desdites au moins deux boucles souples au niveau d'une première extrémité du dispositif médical et l'agencement d'une seconde boucle desdites au moins deux boucles souples au niveau d'une seconde extrémité du dispositif médical.

14. Procédé selon la revendication 10, comprenant en outre le repliement de la boucle souple vers la tige de cathéter en réponse au repliement du dispositif médical vers la configuration d'administration contre la tige de cathéter.
